(19) Europäisches Patentamt
European Patent Office
Office européen des brevets

(11) Veröffentlichungsnummer: **0 039 818**
**A1**

(12) # EUROPÄISCHE PATENTANMELDUNG

(21) Anmeldenummer: 81103152.5

(22) Anmeldetag: 27.04.81

(51) Int. Cl.³: C 07 D 277/68
C 07 D 513/04, A 61 K 31/425

(30) Priorität: 10.05.80 DE 3017977

(43) Veröffentlichungstag der Anmeldung:
18.11.81 Patentblatt 81/46

(84) Benannte Vertragsstaaten:
AT BE CH DE FR GB IT LI LU NL SE

(71) Anmelder: Dr. Karl Thomae GmbH
Postfach 720
D-7950 Biberach (Riss)(DE)

(72) Erfinder: Engel, Wolfhard, Dr. Dipl.-Chem.
Mozartstrasse 13
D-7950 Biberach 1(DE)

(72) Erfinder: Trummlitz, Günter, Dr. Dipl.-Chem.
Buchenweg 27
D-7951 Warthausen 1(DE)

(72) Erfinder: Eberlein Wolfgang, Dr. Dipl.-Chem.
Obere Au 6
D-7950 Biberach 1(DE)

(72) Erfinder: Schmidt, Günther, Dr. Dipl.-Chem.
Johann-Sebastian-Bach-Strasse 27
D-7950 Biberach 1(DE)

(72) Erfinder: Engelhardt, Günther, Prof. Dr.
Unterer Bühl 18
D-7950 Biberach 1(DE)

(72) Erfinder: Zimmermann, Rainer, Dr. Dipl.-Biochem.
Laurenbühlstrasse 17
D-7951 Mittelbiberach(DE)

(54) **Neue substituierte 2(3H)-Benzothiazolone, Verfahren zu ihrer Herstellung, ihre Verwendung zur Herstellung von Arzneimitteln, und Arzneimittel, die diese Verbindungen enthalten.**

(57) Beschrieben werden neue, in 4-, 5-, 6- und/oder 7-Stellung mono- oder disubstituierte 2(3H)-Benzothiazolone der allgemeinen Formel

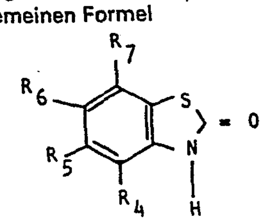
,(I)

worin einer oder zwei der Reste $R_4$, $R_5$, $R_6$ und $R_7$ eine Methyl-, Methoxy-, die Methylenoxy- oder Ethylenoxygruppe, einer dieser Reste auch ein Chloratom oder die Dimethylaminogruppe und die übrigen Reste Wasserstoffatome bedeuten, des weiteren 5 Verfahren zu ihrer Herstellung und Arzneimittel, die eine oder mehrere dieser Verbindungen enthalten.

Die Verbindungen besitzen eine gute analgetische und antipyretische Wirkung und lassen sich zur Herstellung von Arzneimitteln verwenden.

EP 0 039 818 A1

COMPLETE DOCUMENT

Croydon Printing Company Ltd.

0039818

Case 5/791
Dr.Bu/st
Auslandstext

DR. KARL THOMAE GMBH, BIBERACH AN DER RISS

Neue substituierte 2(3H)-Benzothiazolone, Verfahren zu ihrer Herstellung, ihre Verwendung zur Herstellung von Arzneimitteln, und Arzneimittel, die diese Verbindungen enthalten

Die Erfindung betrifft in 4-, 5-, 6- und/oder 7-Stellung mono- oder disubstituierte 2(3H)-Benzothiazolone der allgemeinen Formel I

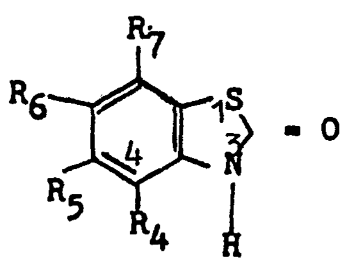

,(I)

In der allgemeinen Formel I bedeuten entweder

$R_4$ die Methylgruppe, $R_6$ die Methoxygruppe, $R_5$ und $R_7$ Wasserstoffatome oder

$R_4$ und $R_6$ jeweils die Methylgruppe, $R_5$ und $R_7$ Wasserstoffatome oder

$R_4$ die Methoxygruppe, $R_5$, $R_6$ und $R_7$ Wasserstoffatome oder

$R_4$ die Methoxygruppe, $R_5$ das Chloratom, $R_6$ und $R_7$ Wasserstoffatome oder

$R_5$ und $R_6$ zusammen die Methylendioxygruppe, $R_4$ und $R_7$ Wasserstoffatome oder

$R_7$ die Methylgruppe, $R_4$, $R_5$ und $R_6$ Wasserstoffatome oder

$R_5$ und $R_6$ jeweils die Methylgruppe, $R_4$ und $R_7$ Wasserstoffatome oder

$R_6$ und $R_7$ jeweils die Methylgruppe, $R_4$ und $R_5$ Wasserstoffatome oder

$R_6$ die Dimethylaminogruppe, $R_4$, $R_5$ und $R_7$ Wasserstoffatome oder

$R_5$ das Chloratom, $R_6$ die Methoxygruppe, $R_4$ und $R_7$ Wasserstoffatome oder

$R_4$ und $R_6$ jeweils die Methoxygruppe, $R_5$ und $R_7$ Wasserstoffatome oder

$R_5$ die Methoxygruppe, $R_6$ die Methylgruppe, $R_4$ und $R_7$ Wasserstoffatome oder

$R_5$ und $R_6$ zusammen die Ethylendioxygruppe, $R_4$ und $R_7$ Wasserstoffatome oder

$R_6$ und $R_7$ zusammen die Ethylendioxygruppe, $R_4$ und $R_5$ Wasserstoffatome.

Die Verbindungen sind neu. Sie zeichnen sich durch gute analgetische und antipyretische Wirkungsqualitäten bei geringer akuter Toxizität aus.

Die Verbindungen der allgemeinen Formel I lassen sich nach folgenden Methoden herstellen:

1) Ein 2-Amino-thiophenol der allgemeinen Formel II

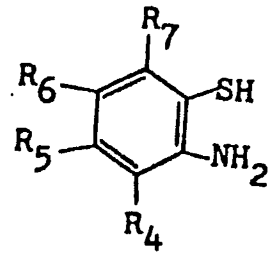

,(II)

in der $R_4$, $R_5$, $R_6$ und $R_7$ wie oben definiert sind, wird mit einem Kohlensäurederivat der allgemeinen Formel III,

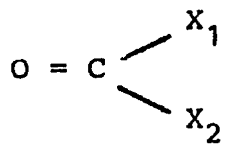

,(III)

in der $X_1$ und $X_2$ gleich oder verschieden sind und Halogen, insbesondere Chlor; die 1-Imidazolyl-; 1,2,4-Triazol-4-yl-; oder die 1,2,4-Triazol-1-ylgruppe;

eine Alkoxygruppe mit 1 bis 10 Kohlenstoffatomen, wie die Methoxy-, Ethoxy-, Propoxy-, Isopropoxy-, Butoxy-, Pentyloxygruppe; die Phenoxy- oder p-Tolyloxygruppe; die Benzyloxy-, Phenethyloxygruppe oder zusammen ein Schwefelatom bedeuten, bei Temperaturen zwischen 0 und 150°C, vorzugsweise zwischen 20°C und der Siedetemperatur des verwendeten Lösungsmittels, und gegebenenfalls in Gegenwart zusätzlicher anorganischer oder organischer Basen, z.B. von Alkali- oder Erdalkalihydroxiden, -carbonaten, -hydrogencarbonaten oder -alkoholaten, von tert. Aminen wie Pyridin, Triethylamin, N,N-Dimethylanilin, N,N-Diethylanilin oder von quartären Ammoniumhydroxiden, z.B. von Benzyltrimethylammoniumhydroxid, cyclisiert.

Als Lösungsmittel eignen sich Wasser, Alkohole wie Methanol, Ethanol, 1-Propanol, 2-Propanol; bevorzugt werden jedoch wasserfreie aprotische Lösungsmittel wie Benzol,

Toluol oder andere Kohlenwasserstoffe, Tetrahydrofuran, 1,4-Dioxan oder andere cyclische Ether, Dimethylformamid, Dimethylsulfoxid. Auch Gemische der genannten Lösungsmittel können verwendet werden.

In einer Variante dieses Verfahrens verwendet man als Kohlensäurederivate Chlorkohlensäureester der allgemeinen Formel IIIa,

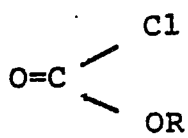

(IIIa)

in der R einen Alkylrest mit 1 bis 10 Kohlenstoffatomen, beispielsweise die Methyl-, Ethyl-, 1-Propyl-, 2-Propyl-, 1-Butyl-, 2-Methyl-1-propyl-, Dimethylethyl-, 1-Pentyl- oder 1-Hexylgruppe, oder die Phenyl-, 4-Methylphenyl-, Phenylmethyl-, 2-Phenylethylgruppe bedeutet; dabei entstehen zunächst Zwischenverbindungen der allgemeinen Formel IV,

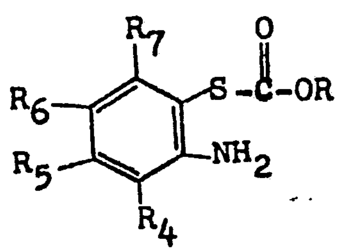

,(IV)

in der R, $R_4$, $R_5$, $R_6$ und $R_7$ wie oben definiert sind. Diese Zwischenverbindungen werden anschließend in Gegenwart saurer Katalysatoren zu den gesuchten Verbindungen der allgemeinen Formel I cyclisiert. Als saure Katalysatoren werden starke oder mittelstarke Mineralsäuren bevorzugt, z.B. Salzsäure, Bromwasserstoffsäure, Schwefelsäure, Phosphorsäure. Die Cyclisierungsreaktion wird in der Regel in inerten organischen Lösungsmitteln duchgeführt, beispielsweise in Methanol, Ethanol, Toluol, Essigsäure, Propionsäure. Die Reaktionstemperaturen liegen

0039818

zwischen 20 und 150°C, vorzugsweise beim Siedepunkt des verwendeten Lösungsmittels.

2) Ein in 2-Stellung durch Chlor, Brom oder Alkoxy substituiertes Benzothiazol der allgemeinen Formel V,

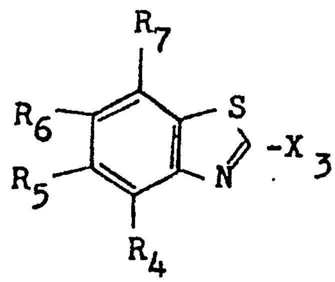

,(V)

in der $R_4$, $R_5$, $R_6$ und $R_7$ wie oben definiert sind und $X_3$ ein Chlor- oder Bromatom oder eine bis zu 3 Kohlenstoffatomen enthaltende Alkoxygruppe bedeutet, wird bei Temperaturen zwischen 0 und 120°C, vorzugsweise bei Siedetemperatur des Reaktionsgemisches, durch Behandlung mit wässerigen Mineralsäuren, beispielsweise mit konz. Salzsäure oder Bromwasserstoffsäure, 20 bis 60%iger Schwefelsäure oder halbkonz. Phosphorsäure, zu einer Verbindung der allgemeinen Formel I hydrolysiert. Die Reaktion kann in Gegenwart inerter, mit Wasser mischbarer Lösungsmittel, z.B. Methanol, Ethanol, 1-Propanol, 2-Propanol, Tetrahydrofuran, 1,4-Dioxan, 1,2-Ethandiol durchgeführt werden, gelingt jedoch auch bei Abwesenheit zusätzlicher Lösungsmittel.

3) o-Nitrophenylthioessigsäuren der allgemeinen Formel VI,

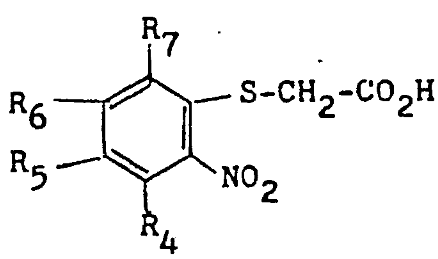

,(VI)

in der $R_4$, $R_5$, $R_6$ und $R_7$ wie eingangs definiert sind, werden durch Erhitzen mit Acetanhydrid- vorzugsweise auf Rückflußtemperatur - zu 3-Acetyl-2(3H)-benzothiazolonen der allgemeinen Formel VII,

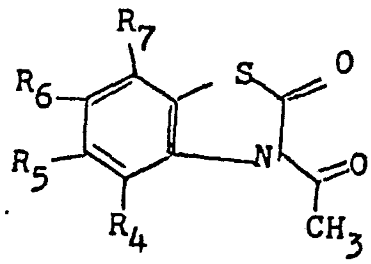

,(VII)

in der $R_4$, $R_5$, $R_6$ und $R_7$ wiederum die eingangs genannten Bedeutungen haben, cyclokondensiert. Die Reaktion wird durch Zusatz von Alkaliacetaten, beispielsweise von Natriumacetat, oder von tertiären organischen Basen, beispielsweise von Pyridin oder Triethylamin katalytisch beschleunigt, gelingt jedoch mit gleicher Ausbeute auch in Abwesenheit von Katalysatoren.

Die Acetylgruppe in einer Verbindung der allgemeinen Formel VII läßt sich durch Umsetzung mit wäßrigen Alkali- oder Erdalkalihydroxiden, z.B. wäßriger Natron-, Kali- oder Barytlauge, mit wäßrigen Aminen oder Ammoniumhydroxid-Lösungen, z.B. konzentriertem Ammoniak oder mit einer Lösung von Benzyltrimethylammoniumhydroxid, oder durch Behandlung mit wäßrigen Mineralsäuren, beispielsweise konzentrierter Salz- oder Bromwasserstoffsäure, halbkonzentrierter Schwefel- oder Phosphorsäure, abspalten unter Bildung der gesuchten Verbindungen der allgemeinen Formel I. Für die Hydrolyse eignen sich Temperaturen zwischen 0 und 120°C, 60 bis 100°C werden jedoch bevorzugt. Die Reaktion kann mit und ohne mit Wasser mischbare Cosolventien durchgeführt werden. In Betracht kommende Lösungsmittel sind beispielsweise

Alkohole, wie Methanol, Ethanol, 2-Propanol, 1,2-Ethandiol, oder Ether, z.B. 1,4-Dioxan oder Tetrahydrofuran.

4) Ein 2(3H)-Benzothiazolthion der allgemeinen Formel VIII,

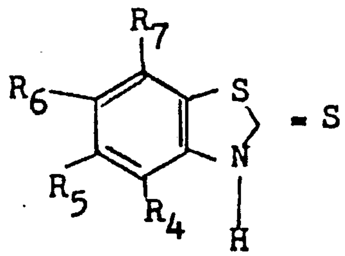

,(VIII)

in der $R_4$, $R_5$, $R_6$ und $R_7$ die eingangs angegebenen Bedeutungen besitzen, wird oxidiert und zu einem 2(3H)-Benzothiazolon der allgemeinen Formel I hydrolysiert. Als Oxidationsmittel werden Brom, Chlor, Wasserstoffperoxid und Alkalipermanganate bevorzugt. Die Reaktion wird in wässerig alkalischem Medium durchgeführt. Als geeignete Basen seien Natriumhydroxid, Kaliumhydroxid, Calciumhydroxid, Bariumhydroxid genannt. Während bei der Oxidation mit Brom, Chlor und Wasserstoffperoxid, die bei -10 bis +30°C durchgeführt wird, Zwischenprodukte nicht nachgewiesen werden können, führt die Umsetzung mit Kaliumpermanganat zunächst zu einer Sulfonsäure der allgemeinen Formel VIIIa,

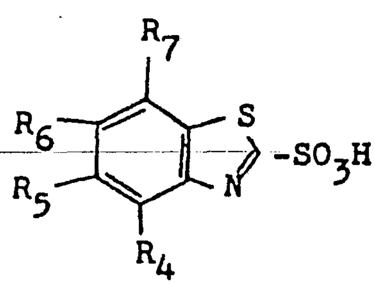

,(VIIIa)

die - in der Regel ohne Isolierung - durch Erwärmen mit wäßrigen Mineralsäuren, beispielsweise mit konz. Salz- oder Bromwasserstoffsäure, mit 30- bis 70%iger Schwefelsäure oder halbkonz. Phosphorsäure, auf Temperaturen zwischen 40 und 100°C in die gesuchten 2(3H)-Benzothiazolone der allgemeinen Formel I übergeführt wird.

Bei der Oxidation mit Brom oder Chlor sollten zur Vermeidung von Kernhalogenierungen keinesfalls mehr als 3 Mol des Halogens pro.Mol 2(3H)-Benzothiazolon der allgemeinen Formel VIII angewendet werden, während die Reaktion mit Wasserstoffperoxid wenigstens 3 Mol, die mit Permanganaten wenigstens 2 Mol des Oxidationsmittels erfordert, wenn eine quantitative Umsetzung angestrebt wird.

In einer Variante dieses Verfahrens werden Alkalisalze von 2(3H)-Benzothiazolthionen der allgemeinen Formel VIII zunächst mit Alkyljodiden oder Dialkylsulfaten oder Benzylhalogeniden zu 2-substituierten Benzothiazolen der allgemeinen Formel IXa,

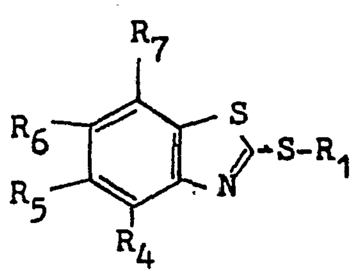

(IXa)

in der $R_4$, $R_5$, $R_6$, $R_7$, wie eingangs definiert sind, und $R_1$ einen niederen Alkylrest mit 1 bis 3 Kohlenstoffatomen oder einen Benzylrest bedeutet, umgesetzt, anschließend mit Kaliumpermanganat in alkalischer Lösung oder in Eisessig zu Sulfonen der allge-

meinen Formel IXb,

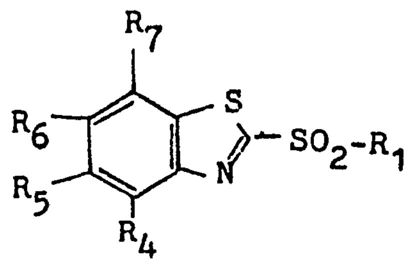

,(IXb)

oxidiert, schließlich durch Erwärmung auf 40 bis 100°C mit wäßrigen Mineralsäuren in Verbindungen der allgemeinen Formel I übergeführt. Die Hydrolyse der Sulfone der allgemeinen Formel IXb wird bevorzugt in Gegenwart von mit Wasser mischbaren Cosolventien, z.B. von Methanol, Ethanol, 1-Propanol, 2-Propanol, 1,4-Dioxan, Tetrahydrofuran durchgeführt, gelingt jedoch auch in Abwesenheit zusätzlicher Lösungsmittel.

5) Zur Herstellung der Verbindung der allgemeinen Formel I, in der $R_6$ die Dimethylaminogruppe, $R_4$, $R_5$ und $R_7$ Wasserstoffatome bedeuten, kann man auch das vorbekannte 6-Amino-2(3H)-benzothiazolon (Jacobson und Kwaysser, Liebigs Ann. Chem. 277, 249 /1893/) mit Formaldehyd und Ameisensäure bei Temperaturen von 60 bis 100°C, vorzugsweise 70-90°C, methylieren. Formaldehyd und Ameisensäure werden jeweils in einer Menge von wenigstens 2 Mol pro Mol 6-Amino-2(3H)-benzothiazolon verwendet. Die Reaktion wird in der Regel ohne zusätzliche Lösungsmittel durchgeführt; die Umsetzung in wäßriger Ameisensäure, wie sie bei Verwendung von wäßriger 85%iger Ameisensäure und 40%iger Formalinlösung als Reagentien - jeweils im Überschuß - anfällt, hat sich jedoch besonders bewährt.

Die als Ausgangsmaterialien erforderlichen 2-Amino-benzenthiole der allgemeinen Formel II lassen sich in Analogie zu literaturbekannten Verfahren herstellen. So erhält man z.B. 2-Amino-4-chlor-5-methoxybenzenthiol vom Fp. 123-125°C (Diisopropylether) durch Hydrolyse von 5-Chlor-6-methoxy-2-benzothiazolamin mit siedender wäßriger Kalilauge (analog R.L. Mital und S.K. Jain, J. chem. Soc. (C) 1969, 2148 bzw. S.K. Jain und R.L. Mital, Z. Naturforsch. 32B, 821 [1977] bzw. H. Hauser, Helv. Chim. Acta 11, 208 [1928] bzw. R. Schuloff u.a., Ber. dtsch. chem. Ges. 61, 2541 [1928]). Entsprechend stellt man 2-Amino-3-methoxy-benzenthiol (Fp. des Hydrochlorids 141-142°C) aus 4-Methoxy-2-benzothiazolamin her.

Die 2-Benzothiazolamine ihrerseits sind bekannt oder können in Analogie zu literaturbekannten Verfahren synthetisiert werden (z.B: H.P. Kaufmann u.a., Arch. Pharm. 273, 31 [1935]; H.P. Kaufmann, Ber. dtsch. chem. Ges. 62B, 390 [1929]; I.G. Farbenindustrie AG/Erf. H.P. Kaufmann und W. Schubert, Deutsches Reichspat. 493 025 vom 08.04.1927; C.A. 24, 2754 [1930]; R.L. Mital und S.K. Jain, J. chem. Soc. (C) 1969, 2148-2149).

Die Ausgangsmaterialien der allgemeinen Formeln III und IIIa sind bekannt.

Die Ausgangsmaterialien der allgemeinen Formel V, in der $X_3$ ein Chlor- oder Bromatom bedeutet, lassen sich in Analogie zu Angaben von G. Mazzone und G. Pappalardo, Il Farmaco, Ed. Sc. 32, 348-354 (1977) aus entsprechenden 2-Benzothiazolaminen durch Umsetzung mit warmer konz. Halogenwasserstoffsäure und Natriumnitrit bereiten.

Die Ausgangsmaterialien der allgemeinen Formel **V**, in der $X_3$ eine Alkoxygruppe ist, sind aus entsprechenden N-Aryl-thiourethanen durch Umsetzung mit heißer wäßrig-alkoholischer Lösung von Kaliumhexacyanoferrat (III) und Natriumhydroxid (analog R.F. Hunter und E.R. Parken, J. chem. Soc. (London) 1935, 1755-1761) oder aus Verbindungen der allgemeinen Formel V, in der $X_3$ ein Chlor- oder Bromatom bedeutet, durch Reaktion mit Alkali- oder Erdalkalialkanolaten, vorzugsweise in den entsprechenden siedenden Alkoholen, zugänglich.

So wurden z.B. hergestellt:
2-Ethoxy-6-methoxy-4-methylbenzothiazol (23 % der Theorie), Fp. 56-57°C (Methanol) aus 4-Methoxy-2-methylphenyl)-thiocarbaminsäure-O-ethylester, Fp. 91°C (Ethanol);
4,6-Dimethyl-2-ethoxybenzothiazol (45 % der Theorie; ohne Reinigung weiterverarbeitet) aus (2,4-Dimethylphenyl)-thiocarbaminsäure-O-ethylester, Fp. 40,5-41,5°C (Benzin);
2-Ethoxy-4-methoxybenzothiazol, Fp. 62-63°C (Petrolether) aus (2-Methoxyphenyl)-thiocarbaminsäure-O-ethylester, Fp. 63-64°C (Diisopropylether);
5-Chlor-2-ethoxy-4-methoxybenzothiazol (27 % der Theorie; nicht kristallisierendes Öl) aus (3-Chlor-2-methoxyphenyl)-thiocarbaminsäure-O-ethylester, Fp. 67-68°C (Methanol);
2-Ethoxy-5,6-methylendioxybenzothiazol (90 % der Theorie; ohne Reinigung in die nächste Stufe eingesetzt) aus (3,4-Methylendioxyphenyl)-thiocarbaminsäure-O-ethylester, Fp.96°C (Petrolether);
2-Ethoxy-7-methylbenzothiazol (Öl) im Gemisch mit 2-Ethoxy-5-methylbenzothiazol aus (3-Methylphenyl)-thiocarbaminsäure-O-ethylester, Fp. 66-67°C (Benzin/Aktivkohle);
2-Ethoxy-5,6-dimethylbenzothiazol im Gemisch mit 2-Ethoxy-6,7-dimethylbenzothiazol (Öl) aus (3,4-Dimethylphenyl)-thiocarbaminsäure-O-ethylester, Fp. 82-84°C (Benzin/Aktivkohle).

2-Ethoxy-6,7-ethylendioxybenzothiazol (43 % der Theorie; Fp. 99-100°C, aus Petrolether) und 2-Ethoxy-5,6-ethylendioxybenzothiazol (21 % der Theorie; nicht kristallisierendes Öl) aus (3,4-Ethylendioxyphenyl)-thiocarbaminsäure-O-ethylester (Fp. 85°C, aus Benzin).
2,5-Dimethoxy-6-methylbenzothiazol (95 % der Theorie) aus 2-Chlor-5-methoxy-6-methylbenzothiazol (Fp. 101°C, aus Petrolether).

2,4,6-Trimethoxybenzothiazol (83 % der Theorie; nicht kristallisierendes Öl) aus 2-Chlor-4,6-dimethoxybenzothiazol. Die erforderlichen N-Aryl-thiourethane erhält man aus - in der Regel - bekannten N-Aryl-isothiocyanaten und wasserfreien Alkoholen in Gegenwart von Chinolin (analog R.F. Hunter und E.R. Parken, J. chem. Soc. (London) 1935, 1755-1761).

Die als Ausgangsmaterialien erforderlichen o-Nitrophenylthioessigsäuren der allgemeinen Formel VI stellt man beispielsweise aus entsprechenden o-Halogen-nitrobenzolen und Thioglykolsäure entweder in siedender wäßrig-alkoholischer Natriumhydroxid-Lösung (analog A.F. Aboulezz, S.M.A. Zayed, W.S. El-Hamouly und M.J. El-Sheikh, Egypt. J. Chem. 16, 355-359 /1973/) oder vorteilhaft in wasserfreiem Dimethylsulfoxid und in Gegenwart von Natriummethylat her.

Die als Ausgangsmaterialien benötigten 2(3H)-Benzothiazolthione der allgemeinen Formel VIII erhält man in Analogie zu Literaturangaben (L.B. Sebrell und C.E. Boord, J. Amer. chem. Soc. 45, 2390-2399 /1923/; J. Teppema und L.B. Sebrell, J. Amer. chem. Soc. 49, 1748-1758 /1927/) beispielsweise aus geeigneten Anilinen, Schwefelkohlenstoff und Schwefel unter Druck und bei erhöhter Temperatur oder aus geeigneten o-Halogennitrobenzolen, Natriumsulfid, Schwefelwasserstoff und Schwefelkohlenstoff.
So erhält man z.B.:

4,6-Dimethyl-2(3H)-benzothiazolthion, Fp. 250-251 °C (gelbe Kristalle aus Ethanol);
7-Methyl-2(3H)-benzothiazolthion, Fp. 184 °C (Anilin);
6-Methoxy-4-methyl-2(3H)-benzothiazolthion, umgesetzt ohne weitere Reinigung.

Die Verbindungen der allgemeinen Formel I stellen sehr gut analgetisch und antipyretisch wirksame Substanzen dar, die weitgehend untoxisch sind.

Es wurden beispielsweise die Substanzen

| | |
|---|---|
| 6-Methoxy-4-methyl-2(3H)-benzothiazolon | = A |
| 4,6-Dimethyl-2(3H)-benzothiazolon | = B |
| 5-(und 7-)Methyl-2(3H)-benzothiazolon | = D |
| 6-Dimethylamino-2(3H)-benzothiazolon | = E |
| 5-Chlor-2,3-dihydro-6-methoxy-2-oxo-benzothiazol | = F |
| 4-Methoxy-2(3H)-benzothiazolon | = G |
| und | |
| 5-Chlor-4-methoxy-2(3H)-benzothiazolon | = H |

auf eine analgetische Wirkung gegenüber dem Entzündungsschmerz der Ratte, eine analgetische Wirkung gegen den durch Wärme ausgelösten Schmerz der Maus, eine die Körpertemperatur der Ratte senkende Wirkung sowie die akute Toxizität an der Maus untersucht.

Methodik

1) Wirkung gegen den Entzündungsschmerz der Ratte

Die Versuchsanordnung entsprach der von RANDALL-SELITTO (Arch. int. Pharmacodyn. 111, 409 (1957) angegebenen.

Männliche Chbb:THOM-Ratten mit einem Gewicht zwischen 100 und 130 g erhielten eine subplantare Injektion von 0,1 ml einer 5%igen Suspension von Hefezellen in 5,55 %iger Glukoselösung in eine Hinterpfote. 1 1/2 Stunden bzw. 2 1/4 Stunden nach der Injektion des Phlogisticums erhielten die Tiere verschiedene Dosen der Prüfsubstanz als Verreibung in 1%iger Methylzellulose (1 ml/100 g Tier) per Schlundsonde beigebracht. Kontrolltiere erhielten entsprechende Volumina des Vehikels.
90 bzw. 45 min. nach Applikation der Prüfsubstanz (d.h. 3 Stunden nach der subplantaren Injektion der Hefesuspension) wurde bei den Ratten die Schmerzschwelle in g Auflagedruck/Pfote bestimmt.

Aus der nach den verschiedenen Dosen der Prüfsubstanz gemessenen Schmerzschwelle wurde nach linearer Regressionsanalyse nach LINDER (Statistische Methoden, 4. Aufl. pp. 148-162, Birkhäuser, Basel 1964) eine $ED_{50}$ mit den Vertrauensgrenzen nach FIELLER (Quart. J. Pharm. Pharmacol. 17, 117 (1944)) als jene Dosis ermittelt, die eine Anhebung der Schmerzschwelle um 50 % gegenüber der der Kontrollen bewirkte.

2) Wirkung gegen den Wärmeschmerz der Maus

Die Prüfung erfolgte mit Hilfe einer Modifikation der Versuchsanordnung nach CHEN u. BECKMAN (Science 113, 631 (1951) an männlichen Chbb:NMRI(SPF)-Mäusen mit einem mittleren Gewicht von 20 g. Die "heiße Platte" bestand aus Aluminium und hatte eine Oberflächentemperatur von 52°C.

Die Prüfsubstanzen wurden als Verreibung in 1%iger Methylzellulose (0,1 ml/10 g Maus) per Schlundsonde beigebracht. Vor der Behandlung mit Prüfsubstanz wurden die Tiere im Abstand von 30 min. zweimal auf die heiße Platte gesetzt. Es wurde ihre individuelle Reaktionszeit ermittelt. Nach Gabe der Prüfsubstanz wurde in Abständen von 30 min. die Reaktionszeit erneut gemessen.

Aus der mit den verschiedenen Dosen der Prüfsubstanzen erzielten gemittelten maximalen Verlängerung der Reaktionszeit wurde nach linearer Regressionsanalyse nach LINDER (s.o.) eine $ED_{100}$ mit den Vertrauensgrenzen nach FIELLER (s.o.) als die Dosis berechnet, die zu einer 100%igen Verlängerung der Reaktionszeit führte.

3) Wirkung auf die Körpertemperatur der Ratte

Die Prüfung der temperatursenkenden Wirkung erfolgte durch die Kontrolle des Verlaufes der rektal gemessenen Körpertemperatur von Chbb:THOM-Ratten mit einem Gewicht zwischen 125-150 g über im Rektum verweilende Thermoelemente. Die Prüfsubstanzen wurden als Verreibung in 1 %iger Methylzellulose (1,0 ml/100 g Tier) per Schlundsonde beigebracht.
Aus den nach Gabe der verschiedenen Dosen der Prüfsubstanz gewonnenen Werten für die mittlere maximale Temperatursenkung wurde nach linearer Regressionsanalyse nach LINDER (s.o.) eine $ED_{-1,5^{o}C}$ als jene Dosis berechnet, die eine Senkung der Körpertemperatur um 1,5°C bewirkte.

4) Akute Toxizität an der Maus

Die Bestimmung der akuten Toxizität erfolgte an Chbb:NMRI (SPF)-Mäusen beider Geschlechter mit einem mittleren Gewicht von 20 g. Die Prüfsubstanzen wurden als Verreibung in 1%iger Methylzellulose (0,5 ml/10 g Tier) per Schlundsonde verabfolgt. Die Berechnung der $LD_{50}$ erfolgte nach LITCHFIELD u. WILCOXON (J. Pharmacol. exp. Therap. 96, 99 (1949)) aus dem Prozentsatz der Tiere, die nach den verschiedenen Dosen innerhalb von 14 Tagen verstarben.

Ergebnisse

Die bei diesen Prüfungen erzielten Befunde sind in den Tabellen 1 bis 5 zusammengefaßt.

Tabelle 1

Wirkung gegen den Entzündungsschmerz der Ratte in der Versuchsanordnung nach RANDALL u. SELITTO 45 min nach oraler Gabe

| Substanz | $ED_{50}$ mg/kg | Vertrauensgrenzen bei 95 % Wahrscheinlichkeit |
|---|---|---|
| A | 28 | 27-30 |
| B | 61 | 57-67 |
| C | 53 | 50-58 |
| D | 40 | 37-43 |
| E | 106 | 98-116 |
| F | 109 | 102-117 |
| G | 105 | 100-110 |
| H | 46 | 45-48 |

Tabelle 2

Wirkung gegen den Entzündungsschmerz der Ratte in der Versuchsanordnung nach RANDALL u. SELITTO 90 min nach oraler Gabe

| Substanz | $ED_{50}$ mg/kg | Vertrauensgrenzen bei 95 % Wahrscheinlichkeit |
|---|---|---|
| A | 23 | 22 - 24 |
| B | 50 | 47 - 53 |
| C | 47 | 44 - 50 |
| D | 40 | 37 - 42 |
| E | 122 | 112 - 136 |
| F | 136 | 126 - 148 |
| G | 99 | 95 - 104 |
| H | 45 | 43 - 47 |

Tabelle 3

Wirkung gegen den Wärmeschmerz der Maus auf der "heißen Platte" nach oraler Gabe

| Substanz | $ED_{100}$ mg/kg | Vertrauensgrenzen bei 95 % Wahrscheinlichkeit |
|---|---|---|
| A | 175 | 153 - 203 |
| B | 194 | 99 - 252 |
| C | 339 | 195 - 358 |
| D | 247 | 154 - 303 |
| E | 181 | 135 - 315 |

Tabelle 4

Wirkung auf die Körpertemperatur der normothermen Ratte nach oraler Gabe

| Substanz | $ED_{-1,5°C}$ mg/kg | Vertrauensgrenzen bei 95 % Wahrscheinlichkeit |
|---|---|---|
| A | 110 | 93 - 134 |
| B | 41 | 33 - 48 |
| C | 37 | 31 - 43 |
| D | 80 | 70 - 90 |
| E | 37 | 32 - 45 |
| F | 171 | 139 - 238 |
| G | 161 | 122 - 279 |
| H | 57 | 45 - 79 |

Tabelle 5

Akute Toxizität an der Maus nach oraler Gabe bei einer Nachbeobachtungszeit von 14 Tagen

| Substanz | $LD_{50}$ mg/kg | Vertrauensgrenzen bei 95 % Wahrscheinlichkeit |
|---|---|---|
| A | 9 000 | 5 450 - 14 850 |
| B | 7 000 | 5 260 - 9 310 |
| C | 7 000 1) | |
| F | 1 250 | 1 059 - 1 475 |

1) nach dieser Dosis verstarb 1 von 10 Tieren

Die nachfolgenden Beispiele sollen die Erfindung näher erläutern:

Beispiel 1

4-Methoxy-2(3H)-benzothiazolon

Zu der Lösung von 271,6 g (1,75 mol) 2-Amino-3-methoxy-benzenthiol in 1,5 l wasserfreiem Tetrahydrofuran gibt man unter Rühren und unter Einhaltung einer Temperatur zwischen 45 und 50°C portionsweise 300,0 g (1,87 mol) N,N'-Carbonyldiimidazol und kocht anschließend 30 Minuten unter Rückfluß. Vom Lösungsmittel werden 1,7 l abdestilliert, die verbleibende Lösung wird heiß filtriert und in 3 l Wasser eingerührt. Nach 1/2 Stunde wird das ausgefallene Produkt abgenutscht und mit Wasser gründlich gewaschen. Man kristallisiert aus Methanol unter Verwendung von Aktivkohle um und erhält 91,5 g (29 % der Theorie) an farblosen Kristallen vom Fp. 175-176°C.

$C_8H_7NO_2S$ (181,21)

| | | | | |
|---|---|---|---|---|
| Ber.: | C 53,03 | H 3,89 | N 7,73 | S 17,69 |
| Gef.: | 53,21 | 3,95 | 7,68 | 17,60 |

Beispiel 2

5-Chlor-6-methoxy-2(3H)-benzothiazolon

In die Lösung von 9,5 g (0,o5 mol) 2-Amino-4-chlor-5-methoxy-benzenthiol und 5,0 g Triethylamin (0,05 mol) in 60 ml wasserfreiem Tetrahydrofuran leitet man unter äußerer Kühlung mit Eis insgesamt 3,3 g (0,055 mol) Carbonylsulfid ein, rührt die Mischung anschließend 16 Stunden bei Raumtemperatur und kocht 8 Stunden unter Rückfluß. Nach 72-stündigem Stehenlassen bei Zimmertemperatur nutscht man den Niederschlag

ab und kristallisiert ihn aus Tetrahydrofuran um. Durch Aufarbeitung der Mutterlaugen läßt sich weiteres Material von gleicher Reinheit gewinnen. Insgesamt erhält man 6,0 g (56 % der Theorie) an farblosen Kristallen vom Fp. 260-261°C (Tetrahydrofuran).

$C_8H_6ClNO_2S$ (215,65)

| | | | | | |
|---|---|---|---|---|---|
| Ber.: | C 44,56 | H 2,80 | Cl 16,44 | N 6,49 | S 14,87 |
| Gef.: | 44,52 | 2,85 | 16,38 | 6,51 | 14,95 |

Beispiel 3

5-Chlor-6-methoxy-2(3H)-benzothiazolon

18,97 g (0,1 mol) 2-Amino-4-chlor-5-methoxybenzenthiol werden in 100 ml einer Mischung aus gleichen Volumteilen wasserfreien Tetrahydrofurans und Toluols gelöst, unter Rühren mit 5,0 g (ca. 0,1 mol) 50%igem Natriumhydrid versetzt, nach beendeter Wasserstoffentwicklung auf ca. 0°C gekühlt. Unter äußerer Kühlung mit Eis und unter Einhaltung einer Reaktionstemperatur von 0 bis +5°C tropft man 50 ml (ca. 0,1 mol) einer 20%igen Lösung von Phosgen in Toluol zu, rührt anschließend noch 2 Stunden bei Zimmertemperatur und nutscht den entstandenen Niederschlag ab. Nach zweimaligem Umkristallisieren aus Ethanol, jeweils unter Verwendung von Aktivkohle erhält man 2,1 g ( 10% der Theorie) an farblosen Kristallen vom Fp. 259-260°C, nach Mischschmelzpunkt und dünnschichtchromatographischer Untersuchung identisch mit einem nach Beispiel 2 hergestellen Präparat.

$C_8H_6ClNO_2S$ (215,65)

| | | | | | |
|---|---|---|---|---|---|
| Ber.: | C 44,56 | H 2,80 | Cl 16,44 | N 6,49 | S 14,87 |
| Gef.: | 44,76 | 3,16 | 16,25 | 6,34 | 14,62 |

Beispiel 4

5-Chlor-6-methoxy-2(3H)-benzothiazolon

Zu der eiskalten Lösung von 2,12 g (0,053 mol) Natriumhydroxid in 50 ml Wasser fügt man nacheinander 10,05 g (0,053 mol) 2-Amino-4-chlor-5-methoxybenzenthiol und 1 g einer 40%igen methanolischen Lösung von Benzyltrimethylammoniumhydroxid. Unter Einhaltung einer Reaktionstemperatur von 5 bis 6°C tropft man anschließend innerhalb von 40 Minuten 8,6 g (0,079 mol) Chlorkohlensäurethylester ein, nimmt die Kühlung fort und rührt weitere 30 Minuten bei Raumtemperatur. Der entstandene Niederschlag wird abfiltriert, mit 25 ml Diisopropylether gewaschen und getrocknet. Ausbeute an 5-Chlor-2-ethoxycarbonylthio-4-methoxyanilin: 12,2 g (88 % der Theorie).

8,17 g (0,0312 mol) 5-Chlor-2-ethoxycarbonylthio-4-methoxyanilin werden zu einer Mischung aus 19 ml Methanol und 2,6 ml konz. Schwefelsäure gegeben und 40 Minuten unter Rückfluß gekocht. Man läßt erkalten, rührt die Mischung in 50 ml Eiswasser ein und filtriert. Der Niederschlag wird in der Lösung von 1,7 g Natriumhydroxid in 150 ml Wasser aufgenommen, mit Aktivkohle behandelt und filtriert, worauf man das Filtrat noch zweimal mit 50 ml Ether auszieht. Die wäßrige Phase wird mit 3 N Salzsäure auf pH 7 gebracht, worauf man den entstandenen Niederschlag abnutscht, trocknet und schließlich aus Ethanol umkristallisiert. Man erhält 5,4 g (80 % der Theorie) an farblosen Kristallen vom Fp. 260-261°C, nach Mischschmelzpunkt, Elementaranalyse und IR-Spektrum identisch mit einem nach Beispiel 2 hergestellten Präparat.

Beispiel 5

4-Methoxy-2(3H)-benzothiazolon

a) 3-Acetyl-4-methoxy-2(3H)-benzothiazolon

Die Lösung von 24,5 g (0,1007 mol) /(3-Methoxy-2-nitrophenyl)thio/-essigsäure in 150 ml Acetanhydrid wird 6 Std. unter Rückfluß gekocht. Das überschüssige Acetanhydrid wird im Wasserstrahlvakuum abdestilliert, der verbleibende Rückstand zwischen Wasser und Dichlormethan verteilt. Die Methylenchloridphase wird eingedampft, der Rückstand an Kieselgel unter Verwendung von Dichlormethan/Essigsäureethylester (Volumenverhältnis 1:1) zum Eluieren chromatographisch gereinigt. Das nach dem Eindampfen der Eluate erhaltene kristallisierende Produkt (11,5 g, d.s. 51 % der Theorie) wird ohne weitere Reinigung in die nächste Stufe eingesetzt.

b) 4-Methoxy-2(3H)-benzothiazolon

7,0 g (0,o314 mol) 3-Acetyl-4-methoxy-2(3H)-benzothiazolon werden in einer Mischung aus 210 ml 5 N Salzsäure und 70 ml Ethanol suspendiert und 1 Stunde unter Rückfluß gekocht. Die noch heiße Mischung wird filtriert, mit 200 ml Wasser verdünnt und durch äußere Kühlung mit Eis auf eine Temperatur von +5°C gebracht. Nach 2 stündigem Stehenlassen wird der Niederschlag abgenutscht, das getrocknete Produkt an Kieselgel unter Verwendung von 1,2-Dichlorethan/Aceton (Volumenverhältnis 9:1) zum Eluieren chromatographisch gereinigt, zuletzt aus Methanol und aus 1,2-Dichlorethan, jeweils unter Zusatz von Aktivkohle, umkristallisiert. Die in einer Ausbeute von 2,9 g (51 % der Theorie) erhaltenen Kristalle schmelzen bei 175-176°C.

$C_8H_7NO_2S$ (181,21)

| | | | | |
|---|---|---|---|---|
| Ber.: | C 53,03 | H 3,89 | N 7,73 | S 17,69 |
| Gef.: | 53,10 | 3,94 | 7,78 | 17,78 |

Beispiel 6

4-Methoxy-2(3H)-benzothiazolon

a) 3-Acetyl-4-methoxy-2(3H)-benzothiazolon

Man arbeitet wie in Beispiel 5a), setzt dem Reaktionsansatz jedoch 50,0 g (0,61 mol) wasserfreies Natriumacetat zu. Die Reaktionstemperatur kann dadurch auf 100°C, die Reaktionsdauer auf 30 Minuten gesenkt werden. Die Ausbeute an rohem 3-Acetyl-4-methoxy-2(3H)-benzothiazolon beträgt 11,0 g (49 % der Theorie).

b) 2,223 g (0,01 mol) 3-Acetyl-4-methoxy-2(3H)-benzothiazolon werden in der Mischung aus 40 ml Ethanol und 50 ml konz. Ammoniak 2 Stunden unter Rückfluß gekocht. Man dampft die Mischung auf ein Viertel ihres ursprünglichen Volumens ein, läßt erkalten, filtriert und reinigt den getrockneten Niederschlag durch Chromatographieren an Kieselgel unter Verwendung von 1,2-Dichlorethan/Aceton (Volumenverhältnis 9:1) zum Eluieren. Die eingedampften Eluate werden aus Methanol unter Verwendung von Aktivkohle umkristallisiert und ergeben 1,55 g (85 % der Theorie) des gewünschten Produktes vom Schmelzpunkt 175-176°C, nach Mischschmelzpunkt und Elementaranalyse identisch mit einem nach Beispiel 1 erhaltenen Präparat.

Beispiel 7

6-Methoxy-4-methyl-2(3H)-benzothiazolon

Die Lösung von 12,3 g (55 mmol) 2-Ethoxy-6-methoxy-4-methylbenzothiazol in 250 ml siedendem Ethanol wird tropfenweise

mit 45 ml konzentrierter wäßriger Salzsäure versetzt. Man kocht anschließend 2 Stunden unter Rückfluß und destilliert das Lösungsmittel ab. Der Rückstand wird in Ether aufgenommen, mit Wasser säurefrei gewaschen und abermals zur Trockne eingeengt. Der Rückstand wird aus Ethanol umkristallisiert. Man erhält 7,6 g (70 % der Theorie) an 6-Methoxy-4-methyl-2(3H)-benzothiazolon vom Fp. 204°C.

$C_9H_{10}NO_2S$ (196,25)

| | | | | |
|---|---|---|---|---|
| Ber.: | C 55,36 | H 4,65 | N 7,17 | S 16,42 |
| Gef.: | 55,26 | 4,41 | 7,12 | 16,27 |

IR($CH_2Cl_2$): NH 3420, CO 1700 und 1675 $cm^{-1}$

1H-NMR ($CDCl_3$): 10,0 (1H-s, br, NH); 6,8 (1H-d; J= 1,5Hz); 6,68 (1H-d; J= 1,5Hz); 3,77 (3H-s; $OCH_3$); 2,38 (3H-s; $CH_3$).

## Beispiel 8

### 4,6-Dimethyl-2(3H)-benzothiazolon

Hergestellt analog Beispiel 7 aus 4,6-Dimethyl-2-ethoxybenzothiazol und konzentrierter Salzsäure in einer Ausbeute von 74 % der Theorie. Fp. 219°C (Ethanol).

$C_9H_9NOS$ (179,25)

| | | | | |
|---|---|---|---|---|
| Ber.: | C 60,31 | H 5,06 | N 7,81 | S 17,89 |
| Gef.: | 60,70 | 5,20 | 7,62 | 17,77 |

IR(KBr): NH 3150, CO 1670 $cm^{-1}$

1H-NMR($CDCl_3$ + $CD_3OD$): 7,05 (1H-s,br); 6,88 (1H-s,br); 2,35 (6H-s,br, 4- und 6-$CH_3$).

## Beispiel 9

### 4-Methoxy-2(3H)-benzothiazolon

Hergestellt analog Beispiel 7 aus 2-Ethoxy-4-methoxybenzothiazol und konzentrierter Salzsäure in einer Ausbeute von 69 % der Theorie.

Fp. 175-176°C (Methanol).

$C_8H_7NO_2S$ (181,21)

| | | | | |
|---|---|---|---|---|
| Ber.: | C 53,03 | H 3,89 | N 7,73 | S 17,69 |
| Gef.: | 53,07 | 3,87 | 7,75 | 17,72 |

Beispiel 10

5-Chlor-4-methoxy-2(3H)-benzothiazolon

Hergestellt analog Beispiel 7 aus 5-Chlor-2-ethoxy-4-methoxybenzothiazol und konzentrierter Salzsäure in einer Ausbeute von 33 % der Theorie.
Fp. 191-192°C (Methanol).
$C_8H_6ClNO_2S$ (215,65)

| | | | | | |
|---|---|---|---|---|---|
| Ber.: | C 44,56 | H 2,80 | Cl 16,44 | N 6,49 | S 14,87 |
| Gef.: | 44,71 | 2,90 | 16,33 | 6,80 | 15,05 |

Beispiel 11

5,6-Methylendioxy-2(3H)-benzothiazolon

Hergestellt analog Beispiel 7 aus 2-Ethoxy-5,6-methylendioxybenzothiazol und konzentrierter Salzsäure in einer Ausbeute von 71 % der Theorie.
Fp. 261°C (Ethanol).
$C_8H_5NO_3S$ (195,2)

| | | | | |
|---|---|---|---|---|
| Ber.: | C 49,22 | H 2,58 | N 7,18 | S 16,43 |
| Gef.: | 48,96 | 2,53 | 7,03 | 16,45 |

IR(KBr): NH 3400 und 3180, CO 1670 $cm^{-1}$
1H-NMR ($d_6$-DMSO): 11,5 (1H,br, NH, austauschbar); 7,15 (1H-s); 6,7 (1H-s); 6,00 (2H-s; $OCH_2O$)

Beispiel 12

7-Methyl-2(3H)-benzothiazolon

Hergestellt analog Beispiel 7 aus einem Gemisch von 2-Ethoxy-7-methylbenzothiazol und 2-Ethoxy-5-methylbenzothiazol und konzentrierter Salzsäure in einer Ausbeute von 65 % der Theorie.
Fp. 137-138°C (Ethanol/Wasser 1:1); das Produkt enthält ca. 10 % des isomeren 5-Methyl-2(3H)-benzothiazolon.
$C_8H_7NOS$ (165,21)

| | | | | |
|---|---|---|---|---|
| Ber.: | C 58,16 | H 4,27 | N 8,48 | S 19,41 |
| Gef.: | 58,27 | 4,18 | 8,35 | 19,18 |

Beispiel 13

5,6-Dimethyl-2(3H)-benzothiazolon und 6,7-Dimethyl-2(3H)-benzothiazolon

Aus einem 5,6-Dimethyl-2-ethoxybenzothiazol/6,7-Dimethyl-2-ethoxy-benzothiazol-Gemisch und konzentrierter Salzsäure erhält man analog Beispiel 7 in einer Ausbeute von 28 % der Theorie ein 1:1-Gemisch der beiden Titelverbindungen.
Fp. 168-194°C (Ethanol/Aktivkohle).
$C_9H_9NOS$ (179,24)

| | | | | |
|---|---|---|---|---|
| Ber.: | C 60,31 | H 5,06 | N 7,81 | S 17,89 |
| Gef.: | 60,39 | 5,18 | 7,82 | 17,75 |

Beispiel 14

6-(Dimethylamino)-2(3H)-benzothiazolon

Zu der Mischung aus 27,0 g (0,9 mol) Paraformaldehyd und 67,0 g (1,46 mol) Ameisensäure gibt man in kleinen Portionen und unter Einhaltung einer Reaktionstemperatur von 80 bis 90°C

innerhalb von ca. 40 Minuten 60,0 g (0,361 mol) 6-Amino-2(3H)-benzothiazolon. Anschließend hält man noch 10 Minuten bei 80°C. Die überschüssige Ameisensäure wird im Vakuum abdestilliert, der Rückstand in heißem Acetonitril gelöst und filtriert. Das Filtrat wird eingedampft, der verbleibende Rückstand an Kieselgel unter Verwendung eines Gemisches aus 180 Raumteilen Chloroform, 75 Teilen Esssigsäureethylester, 25 Teilen Cyclohexan, 25 Teilen Methanol und 3 Teilen konzentriertem Ammoniak zum Eluieren säulenchromatographiert. Nach abschließendem Umkristallisieren aus Ethanol erhält man 19,3 g (28 % der Theorie), an farblosen Kristallen vom Fp. 194-196°C.

$C_9H_{10}N_2OS$ (194,25)

| | | | | |
|---|---|---|---|---|
| Ber.: | C 55,65 | H 5,19 | N 14,42 | S 16,50 |
| Gef.: | 55,92 | 5,31 | 14,25 | 16,22 |

Beispiel 15

4,6-Dimethyl-2(3H)-benzothiazolon

In eine Suspension von 17,83 g (0,1 mol) 4,6-Dimethyl-2-benzothiazolamin in 2000 ml konzentrierter wäßriger Salzsäure trägt man unter Einhaltung einer Reaktionstemperatur von ca. 50°C in kleinen Portionen innerhalb von ca. 3 Stunden insgesamt 20,0 g (0,29 mol) Natriumnitrit ein. Man läßt über Nacht bei Zimmertemperatur stehen, nutscht den Niederschlag ab und wäscht ihn gründlich mit Wasser. Man trocknet das Produkt an der Luft und kann es ohne weitere Reinigung in der nächsten Stufe verwenden. Ausbeute an 2-Chlor-4,6-dimethylbenzothiazol: 9,75 g (49 % der Theorie).

3,954 g (0,02 mol) 2-Chlor-4,6-dimethylbenzothiazol werden in 100 ml einer Mischung aus gleichen Teilen Ethanol und konzentrierter Salzsäure 4 Stunden unter Rückfluß gekocht.

Man verdünnt mit 100 ml Wasser, filtriert den erhaltenen Niederschlag ab und wäscht ihn gründlich mit Wasser. Zur weiteren Reinigung nimmt man in 10%iger Natronlauge auf, filtriert und ethert die Filtrate zweimal mit je 100 ml Ether aus. Die wäßrige Phase wird mit Salzsäure angesäuert, der anfallende Niederschlag abgenutscht, mit Wasser gewaschen und einmal aus Ethanol, einmal aus Toluol, jeweils unter Verwendung von Aktivkohle, umkristallisiert. Man erhält 2,85 g (79 % der Theorie) an Kristallen vom Schmelzpunkt 219°C, nach Mischschmelzpunkt, Elementaranalyse und dünnschichtgromatographischer Untersuchung identisch mit einem nach Beispiel 8 erhaltenen Präparat.

Beispiel 16

4,6-Dimethyl-2(3H)-benzothiazolon

229,8 g (1,177 mol) 4,6-Dimethyl-2(3H)-benzothiazolthion werden in eine Lösung von 120 g (3 mol) Ätznatron in 1200 ml Wasser eingetragen und bei einer Reaktionstemperatur zwischen 20 und 30°C unter Rühren mit 340 g (ca. 3 mol) 30%iger Wasserstoffperoxid-Lösung versetzt. Man rührt weitere 2 Stunden bei 30°C, 2 Stunden bei 60°C, läßt erkalten und stellt mit Salzsäure auf pH 6,5. Der Niederschlag wird abfiltriert und zweimal aus Ethanol unter Verwendung von Aktivkohle umkristallisiert. Man erhält 164,3 g (92 % der Theorie) an Kristallen vom Fp. 219-220°C, nach Mischschmelzpunkt, Elementaranalyse und IR-Spektrum identisch mit einem nach Beispiel 8 hergestellten Präparat.

Beispiel 17

7-Methyl-2(3H)-benzothiazolon

In eine Lösung von 32,0 g (0,8 mol) Natriumhydroxid und 18,13 g (0,1 mol) 7-Methyl-2(3H)-benzothiazolthion in 400 ml

Wasser leitet man unter Einhaltung einer Temperatur zwischen -10 und + 10°C 21,3 g (0,3 mol) gasförmiges Chlor ein. Man filtriert den Niederschlag ab, wäscht ihn gründlich mit Wasser aus und erhält nach dem Umkristallisieren aus Ethanol unter Verwendung von Aktivkohle 14,5 g (88 % der Theorie) an Kristallen vom Fp. 169-170°C.

$C_8H_7NOS$ (165,21)

| | | | | |
|---|---|---|---|---|
| Ber.: | C 58,16 | H 4,27 | N 8,48 | S 19,41 |
| Gef.: | 58,30 | 4,16 | 8,52 | 19,60 |

Das Produkt ist nach dünnschichtgromatographischer Untersuchung identisch mit der Hauptkomponente des nach Beispiel 12 erhaltenen Präparats.

## Beispiel 18

### 4,6-Dimethyl-2(3H)-benzothiazolon

9,765 g (0,05 mol) 4,6-Dimethyl-2(3H)-benzothiazolthion werden mit 100 ml einer 1 N Natriumhydroxid-Lösung verrieben, filtriert und nach Zugabe einer Lösung von 4,0 ml (9,12 g, d.s. 0,064 mol) Methyljodid in 10 ml Ethanol 15 Minuten bei Zimmertemperatur geschüttelt. Nach 1-stündigem Stehenlassen filtriert man das Reaktionsprodukt ab, wäscht es mit Wasser und trocknet es bei 50°C, Ausbeute an farblosem Produkt: 6,6 g (63 % der Theorie).

5,23 g (0,025 mol) 4,6-Dimethyl-2-methylthiobenzothiazol werden ohne weitere Reinigung in 40 ml Eisessig gelöst. Unter Rühren tropft man innerhalb von 30 Minuten die Lösung von 8,25 g (0,052 mol) Kaliumpermanganat in 125 ml Wasser ein. Durch äußere Kühlung mit Eis sorgt man dafür, daß die Temperatur der Reaktionsmischung in dieser Phase 35°C nicht übersteigt. Man rührt noch 1 Stunde bei Raumtemperatur, läßt erkalten und leitet unter äußerer Kühlung mit Eis Schwefeldioxid durch die Mischung, bis die braune Färbung verschwunden ist. Das

entstandene Sulfon wird abfiltriert und an der Luft getrocknet Ausbeute: 4,9 g (81 % der Theorie).

Das erhaltene 4,6-Dimethyl-2-methylsulfonylbenzothiazol wird ohne weitere Reinigung weiterverarbeitet. Man kocht die Lösung von 4,82 g (0,02 mol) 4,6-Dimethyl-2-methylsulfonylbenzothiazol in 40 ml konzentrierter wäßriger Salzsäure und 4 ml Ethanol 4 Stunden unter Rückfluß, verdünnt mit 200 ml Wasser und stellt die Mischung nach dem Erkalten durch Zugabe von 10 N Natriumhydroxid-Lösung alkalisch. Man filtriert, säuert das Filtrat mit Essigsäure an und nutscht den Niederschlag ab. Man wäscht gründlich mit Wasser, trocknet das Produkt an der Luft und kristallisiert zweimal aus Ethanol um. Man erhält 2,3 g (64 % der Theorie) an farblosen Kristallen vom Fp. 219-220$^{o}$C, die nach Mischschmelzpunkt, Elementaranalyse und dünnschichtchromatographischer Untersuchung völlig mit einem nach Beispiel 8 erhaltenen Produkt übereinstimmen.

Beispiel 19

5,6-Dimethyl-2(3H)-benzothiazolon

Hergestellt analog Beispiel 1 aus 2-Amino-4,5-dimethylbenzenthiol und N,N'-Carbonyldiimidazol in einer Ausbeute von 72 % der Theorie.
Fp. 226-228$^{o}$C (Chloroform/Methanol 1:1).
$C_9H_9NOS$ (179,25)

| | | | | |
|---|---|---|---|---|
| Ber.: | C 60,31 | H 5,06 | N 7,81 | S 17,89 |
| Gef.: | 60,01 | 5,09 | 8,06 | 17,85 |

Beispiel 20

6,7-Ethylendioxy-2(3H)-benzothiazolon

Hergestellt analog Beispiel 7 aus 2-Ethoxy-6,7-ethylendioxy-benzothiazol und konzentrierter Salzsäure in einer Ausbeute von 74 % der Theorie.
Fp. 245°C (Ethanol)
$C_9H_7NO_3S$ (209,22)

| | | | | |
|---|---|---|---|---|
| Ber.: | C 51,67 | H 3,37 | N 6,69 | S 15,33 |
| Gef.: | 51,95 | 3,35 | 6,93 | 15,38 |

Beispiel 21

5,6-Ethylendioxy-2(3H)-benzothiazolon

Hergestellt analog Beispiel 7 aus 2-Ethoxy-5,6-ethylendioxy-benzothiazol und konzentrierter Salzsäure in einer Ausbeute von 78 % der Theorie.
Fp. 244°C (1,2-Dichlorethan)
$C_9H_7NOS$ (209,22)

| | | | | |
|---|---|---|---|---|
| Ber.: | C 51,67 | H 3,37 | N 6,69 | S 15,33 |
| Gef.: | 51,48 | 3,30 | 6,60 | 15,48 |

Beispiel 22

5-Methoxy-6-methyl-2(3H)-benzothiazolon

a) 2-Chlor-5-methoxy-6-methylbenzothiazol

Zu der eisgekühlten Lösung von 19,43 g (0,1 mol) 5-Methoxy-6-methyl-2-benzothiazolamin in 100 ml Eisessig gibt man unter kräftigem Rühren 25,5 ml (ca. 0,3 mol) konzentrierte Salzsäure so zu, daß das entstehende Hydrochlorid in feindisperser Form anfällt. Unter Einhaltung einer Reaktionstemperatur von +8 bis +10°C wird anschließend durch eine feine Düse eine Lösung von 7,3 g (0,106 mol) Natriumnitrit in 11 ml Wasser in das unterste Drittel der gut gerührten Mischung eingespritzt. Die so erhaltene Diazoniumsalzlösung trägt man unter gutem Rühren und unter äußerer Kühlung mit Eis langsam in eine Mischung von 13,3 g (0,134 mol) Kupfer(I)-chlorid und 55 ml konzentrierter Salzsäure ein, wobei eine Reaktionstemperatur von +5 bis +10°C eingehalten werden soll. Nach dem Abklingen der heftigen Stickstoffentwicklung rührt man noch 2 Stunden bei Zimmertemperatur. Dann rührt man in 500 g gestoßenes Eis ein und versetzt mit der Lösung von 50 g wasserfreiem Natriumacetat in 300 ml Wasser. Man schüttelt dreimal mit je 300 ml Ether aus, wäscht die vereinigten Etherextrakte gründlich mit Wasser, gesättigter Natriumhydrogencarbonaltlösung und Wasser und trocknet sie über Natriumsulfat. Nach dem Abdampfen des Lösungsmittels verbleibt eine schwach bräunlich gefärbte Flüssigkeit, die nach längerem Stehen bei 0 bis 5°C kristallin erstarrt, jedoch ohne weitere Reinigung weiterverarbeitet wird. Ausbeute: 15,17 g (71 % der Theorie).

b) 2,5-Dimethoxy-6-methylbenzothiazol

Die Mischung aus 95,3 g (0,446 mol) 2-Chlor-5-methoxy-6-methylbenzothiazol, 680 ml wasserfreiem Methanol und 44,2 g (0,63 mol) Kaliummethanolat wird 1 Stunde unter Rückfluß und Rühren gekocht. Man destilliert das Methanol weitgehend ab, verdünnt die Mischung mit 1 l Eiswasser und extrahiert dreimal mit je 300 ml Ether. Die vereinigten Etherauszüge hinterlassen nach dem Trocknen und Abdestillieren des Lösungsmittels einen farblosen Rückstand, der nach dem Umkristallisieren aus Petrolether unter Verwendung von Aktivkohle bei 101°C schmilzt.
Ausbeute: 73 % der Theorie.

c) 5-Methoxy-6-methyl-2(3H)-benzothiazolon

Die Lösung von 74,7 g (0,357 mol) 2,5-Dimethoxy-6-methyl-benzothiazol in 100 ml siedendem Ethanol wird tropfenweise mit 35 ml konzentrierter Salzsäure versetzt. Man kocht 2 Stunden unter Rückfluß,destilliert das Lösungsmittel ab und arbeitet wie im Beispiel 7 auf. Man erhält 64,1 g (92 % der Theorie) an Kristallen vom Fp. 223-224°C (Ethanol).

$C_9H_9NO_2S$    (195,25)

| | | | | |
|---|---|---|---|---|
| Ber.: | C 55,36 | H 4,65 | N 7,17 | S 16,42 |
| Gef.: | 55,60 | 4,46 | 7,07 | 16,10 |

Beispiel 23

4,6-Dimethoxy-2(3H)-benzothiazolon

Hergestellt analog Beispiel 22c) aus 2,4,6-Trimethoxybenzothiazol und konzentrierter Salzsäure in einer Ausbeute von 79 % der Theorie.

Fp. 183°C (aus Tetrachlormethan)

$C_9H_9NO_3S$    (211,25)

| | | | | |
|---|---|---|---|---|
| Ber.: | C 51,17 | H 4,29 | N 6,63 | S 15,18 |
| Gef.: | 51,11 | 4,24 | 6,75 | 15,16 |

Die Ausgangsverbindung 2,4,6-Trimethoxybenzothiazol erhält man aus 2-Chlor-4,6-dimethoxybenzothiazol und Natriummethanolat entsprechend Beispiel 22b) in einer Ausbeute von 83 % der Theorie.

Beispiel 24

6-Methoxy-4-methyl-2(3H)-benzothiazolon

In eine Lösung von 31,2 g (0,78 mol) Natriumhydroxid in 400 ml Wasser werden 99,3 g (0,47 mol) 6-Methoxy-4-methyl-2(3H)-benzothiazolthion eingetragen. Innerhalb von 2 bis 3 Stunden wird dann bei 25 bis 30°C eine Kaliumpermanganatlösung, die durch Auflösen von 196 g (1,24 mol) Kaliumpermanganat in 2 l Wasser bei 40°C erhalten wurde, unter Rühren zugetropft. Nach beendeter Zugabe wird auf 80°C erwärmt und danach vom entstehenden Braunstein abgesaugt. Der Filterrückstand wird in 1 l Wasser aufgenommen, auf 80°C erwärmt und heiß filtriert. Die vereinigten wäßrigen Filtrate werden auf 60 bis 70°C erwärmt, mit 200 ml konzentrierter wäßriger Salzsäure versetzt und 6 Stunden unter Rückfluß gekocht. Nach dem Abkühlen des Reaktionsgemisches wird vom Niederschlag, der noch mit 500 ml Eiswasser und mit 300 ml eiskaltem Ethanol gewaschen wird, abfiltriert. Das Rohmaterial wird einmal aus 1,2-Dichlorethan, einmal aus Ethanol umkristallisiert. Man erhält 66,4 g (72 % der Theorie) an Kristallen vom Fp. 204°C, nach Schmelzpunkt, Mischschmelzpunkt und Elementaranalyse identisch mit dem nach Beispiel 7 erhaltenen Produkt.

Die Verbindungen der allgemeinen Formel I lassen sich in die üblichen pharmazeutischen Zubereitungsformen, wie Tabletten, Dragées, Suppositorien, Kapseln oder Säfte einarbeiten. Die Einzeldosierung beträgt hierbei für Erwachsene 20 bis 600 mg, vorzugsweise 50 bis 300 mg, dies entspricht einer Tagesdosierung von 60 bis 1800 mg, vorzugsweise von 180 bis 900 mg.

Die nachfolgenden Beispiele sollen die Herstellung einiger pharmazeutischer Zubereitungsformen verdeutlichen.

Beispiel I

Tabletten mit 50 mg 6-Methoxy-4-methyl-2(3H)-benzothiazolon

Zusammensetzung:
1 Tablette enthält:

| | |
|---|---|
| Wirkstoff | 50,0 mg |
| Milchzucker | 128,0 mg |
| Kartoffelstärke | 40,0 mg |
| Magnesiumstearat | 2,0 mg |
| | 220,0 mg |

Herstellungsverfahren:

Aus Kartoffelstärke wird durch Erwärmen ein 10%iger Schleim hergestellt. Die Wirksubstanz, Milchzucker und die restliche Kartoffelstärke werden gemischt und mit obigem Schleim durch ein Sieb der Maschenweite 1,5 mm granuliert. Das Granulat wird bei 45°C getrocknet, nochmals durch obiges Sieb gerieben, mit Magnesiumstearat vermischt und zu Tabletten verpreßt.

| | |
|---|---|
| Tablettengewicht: | 220 mg |
| Stempel: | 9 mm |

Beispiel II

Dragées mit 50 mg 6-Methoxy-4-methyl-2(3H)-benzothiazolon

Die nach Beispiel I hergestellten Tabletten werden nach bekannten Verfahren mit einer Hülle überzogen, die im wesentlichen aus Zucker und Talkum besteht. Die fertigen Dragées

werden mit Hilfe von Bienenwachs poliert.
Dragéegewicht: 300 mg

Beispiel III

Kapseln mit 60 mg 6-Methoxy-4-methyl-2(3H)-benzothiazolon

Zusammensetzung:
1 Kapsel enthält:

| | |
|---|---|
| Wirkstoff | 60,0 mg |

Herstellungsverfahren:

Der Wirkstoff wird mikronisiert und in Kapseln abgefüllt, letztere werden anschließend verschlossen.

Beispiel IV

Suppositorien mit 60 mg 6-Methoxy-4-methyl-2(3H)-benzothiazolon

Zusammensetzung:
1 Zäpfchen enthält:

| | |
|---|---|
| Wirkstoff | 60,0 mg |
| Zäpfchenmasse (z.B. Witepsol W 45 $^{R}$) | 1 640,0 mg |
| | 1 700,0 mg |

Herstellungsverfahren:

Die feinpulverisierte Wirksubstanz wird in der geschmolzenen und auf 40°C abgekühlten Zäpfchenmasse suspendiert. Man gießt die Masse bei 37°C in leicht vorgekühlte Zäpfchenformen aus.
Zäpfchengewicht: 1,7 g

Beispiel V

Tabletten zu 200 mg 6-Methoxy-4-methyl-2(3H)-benzothiazolon

1 Tablette enthält:

| | |
|---|---|
| Wirksubstanz | 200,0 mg |
| Milchzucker | 120,0 mg |
| Maisstärke | 70,0 mg |
| Polyvinylpyrrolidon | 8,0 mg |
| Magnesiumstearat | 2,0 mg |
| | 400,0 mg |

Herstellung:

Die Wirksubstanz, der Milchzucker und die Maisstärke werden mit einer wäßrigen Lösung von Polyvinylpyrrolidon gleichmäßig befeuchtet, durch ein Sieb mit 2 mm-Maschenweite gesiebt und im Umlufttrockenschrank bei 50°C getrocknet. Nach erneuter Siebung durch 1,5 mm-Maschenweite wird Magnesiumstearat zugemischt und die Mischung zu Tabletten verpreßt.

Tablettengewicht: 400 mg
Durchmesser: 11 mm, rund, biplan, beidseitige Facette und einseitige Teilkerbe.

Beispiel VI

Suppositorien zu 200 mg 6-Methoxy-4-methyl-2(3H)-benzothiazolon

1 Zäpfchen enthält:

| | |
|---|---|
| Wirksubstanz | 0,20 g |
| Hartfett (z.B. Witepsol H 19 oder Witepsol W 45) | 1,50 g |
| | 1,70 g |

Herstellung:

Das Hartfett wird geschmolzen. Bei 38°C wird die gemahlene Wirksubstanz in der Schmelze homogen dispergiert. Es wird auf 35°C abgekühlt und in schwach vorgekühlte Suppositorienformen ausgegossen.
Zäpfchengewicht: 1,7 g.

Beispiel VII

Suspension mit 200 mg 6-Methoxy-4-methyl-2(3H)-benzothiazolon

100 ml Suspension enthalten:

| | | |
|---|---|---|
| Wirksubstanz | | 4,0 g |
| Carboxymethylcellulose | | 0,1 g |
| p-Hydroxybenzoesäuremethylester | | 0,05g |
| p-Hydroxybenzoesäurepropylester | | 0,01g |
| Rohrzucker | | 10,0 g |
| Glycerin | | 5,0 g |
| Sorbitlösung 70 % | | 20,0 g |
| Aroma | | 0,3 g |
| Wasser dest. | ad. | 100,0 ml |

Herstellungsverfahren:

Dest. Wasser wird auf 70°C erhitzt. Hierin wird unter Rühren p-Hydroxybenzoesäuremethylester und -propylester sowie Glycerin und Carboxymethylcellulose gelöst. Es wird auf Raumtemperatur abgekühlt und unter Rühren die Wirksubstanz zugegeben und homogen dispergiert. Nach Zugabe und Lösen des Zuckers, der Sorbitlösung und des Aromas wird die Suspension zur Entlüftung unter Rühren evakuiert.

5 ml Suspension enthalten 200 mg Wirksubstanz.

## P a t e n t a n s p r ü c h e

1) Neue substituierte 2(3H)-Benzothiazolone der allgemeinen Formel I

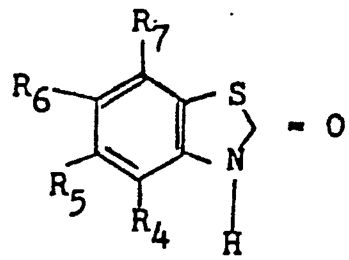

,(I)

in der

entweder $R_4$ die Methylgruppe, $R_6$ die Methoxygruppe, $R_5$ und $R_7$ Wasserstoffatome

oder

$R_4$ und $R_6$ jeweils die Methylgruppe, $R_5$ und $R_7$ Wasserstoffatome oder

$R_4$ die Methoxygruppe, $R_5$, $R_6$ und $R_7$ Wasserstoffatome oder

$R_4$ die Methoxygruppe, $R_5$ das Chloratom, $R_6$ und $R_7$ Wasserstoffatome oder

$R_5$ und $R_6$ zusammen die Methylendioxygruppe, $R_4$ und $R_7$ Wasserstoffatome oder

$R_7$ die Methylgruppe, $R_4$, $R_5$ und $R_6$ Wasserstoffatome oder

$R_5$ und $R_6$ jeweils die Methylgruppe, $R_4$ und $R_7$ Wasserstoffatome oder

$R_6$ und $R_7$ jeweils die Methylgruppe, $R_4$ und $R_5$ Wasserstoffatome oder

$R_6$ die Dimethylaminogruppe, $R_4$, $R_5$ und $R_7$ Wasserstoffatome oder

$R_5$ das Chloratom, $R_6$ die Methoxygruppe, $R_4$ und $R_7$ Wasserstoffatome oder

$R_4$ und $R_6$ jeweils die Methoxygruppe, $R_5$ und $R_7$ Wasserstoffatome oder

$R_5$ die Methoxygruppe, $R_6$ die Methylgruppe, $R_4$ und $R_7$ Wasserstoffatome oder

$R_5$ und $R_6$ zusammen die Ethylendioxygruppe, $R_4$ und $R_7$ Wasserstoffatome oder

$R_6$ und $R_7$ zusammen die Ethylendioxygruppe, $R_4$ und $R_5$ Wasserstoffatome.

2) Als neue Substanz das 6-Methoxy-4-methyl-2(3H)-benzothiazolon.

3) Als neue Substanz das 4,6-Dimethyl-2(3H)-benzothiazolon.

4) Als neue Substanz das 5,6-Methylendioxy-2(3H)-benzothiazolon.

5) Arzneimittel enthaltend eine oder mehrere Substanzen der allgemeinen Formel I gemäß Anspruch 1 neben den üblichen Träger- und/oder Hilfsstoffen.

6) Verwendung von Verbindungen der allgemeinen Formel I gemäß Anspruch 1 zur Herstellung von analgetisch wirkenden Arzneimitteln.

7) Verfahren zur Herstellung von neuen 2(3H)-Benzothiazolonen der allgemeinen Formel I

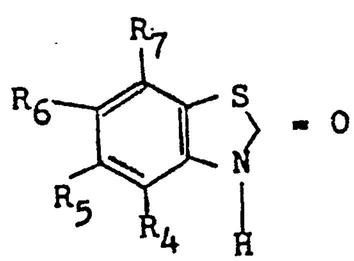

,(I)

in der
entweder
$R_4$ die Methylgruppe, $R_6$ die Methoxygruppe, $R_5$ und $R_7$ Wasserstoffatome oder
$R_4$ und $R_6$ jeweils die Methylgruppe, $R_5$ und $R_7$ Wasserstoffatome oder
$R_4$ die Methoxygruppe, $R_5$, $R_6$ und $R_7$ Wasserstoffatome oder
$R_4$ die Methoxygruppe, $R_5$ das Chloratom, $R_6$ und $R_7$ Wasserstoffatome oder
$R_5$ und $R_6$ zusammen die Methylendioxygruppe, $R_4$ und $R_7$ Wasserstoffatome oder

$R_7$ die Methylgruppe, $R_4$, $R_5$ und $R_6$ Wasserstoffatome oder

$R_5$ und $R_6$ jeweils die Methylgruppe, $R_4$ und $R_7$ Wasserstoffatome oder

$R_6$ und $R_7$ jeweils die Methylgruppe, $R_4$ und $R_5$ Wasserstoffatome oder

$R_6$ die Dimethylaminogruppe, $R_4$, $R_5$ und $R_7$ Wasserstoffatome oder

$R_5$ das Chloratom, $R_6$ die Methoxygruppe, $R_4$ und $R_7$ Wasserstoffatome oder

$R_4$ und $R_6$ jeweils die Methoxygruppe, $R_5$ und $R_7$ Wasserstoffatome oder

$R_5$ die Methoxygruppe, $R_6$ die Methylgruppe, $R_4$ und $R_7$ Wasserstoffatome oder

$R_5$ und $R_6$ zusammen die Ethylendioxygruppe, $R_4$ und $R_7$ Wasserstoffatome oder

$R_6$ und $R_7$ zusammen die Ethylendioxygruppe, $R_4$ und $R_5$ Wasserstoffatome bedeuten,

dadurch gekennzeichnet, daß

a) entweder ein 2-Aminothiophenol der allgemeinen Formel II,

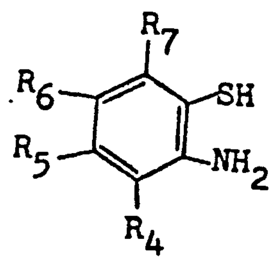

,(II)

in der $R_4$, $R_5$, $R_6$ und $R_7$ wie oben definiert sind, mit einem Kohlensäurederivat der allgemeinen Formel III,

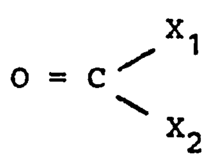

,(III)

in der $X_1$ und $X_2$ gleich oder voneinander verschieden sind und Halogenatome, die 1-Imidazolyl-, 1,2,4-Triazol-4-yl- oder die 1,2,4-Triazol-1-ylgruppe, eine Alkoxygruppe mit 1 bis 10 Kohlenstoffatomen, die Phenoxy- oder p-Tolyloxygruppe, die Benzyloxy- oder Phenethyloxygruppe oder zusammen ein Schwefelatom bedeuten, in einem Lösungsmittel bei Temperaturen zwischen 0 und 150°C umgesetzt wird

oder eine Verbindung der allgemeinen Formel II mit einem Chlorkohlensäureester der allgemeinen Formel IIIa,

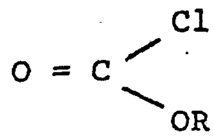

(IIIa)

in der R einen Alkylrest mit 1 bis 10 Kohlenstoffatomen oder die Phenyl-, 4-Methylphenyl-, Phenylmethyl- oder 2-Phenylethylgruppe bedeutet, zur Reaktion gebracht wird, wobei zunächst Verbindungen der allgemeinen Formel IV

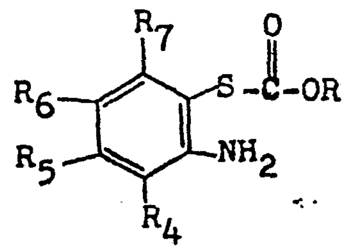

,(IV)

in der R, $R_4$, $R_5$, $R_6$ und $R_7$ wie oben definiert sind, entstehen, die anschließend in Gegenwart saurer Katalysatoren bei Temperaturen zwischen 20 und 150°C zu den Verbindungen der allgemeinen Formel I cyclisiert werden, oder

b) ein in 2-Stellung durch Chlor, Brom oder eine Alkoxygruppe substituiertes Benzothiazol der allgemeinen Formel V,

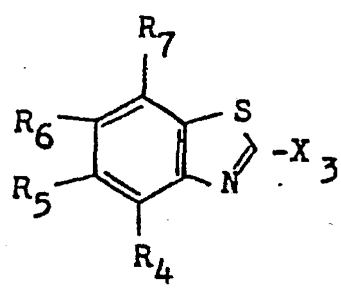

,(V)

in der $R_4$, $R_5$, $R_6$ und $R_7$ wie oben definiert sind und $X_3$ ein Chlor- oder Bromatom oder eine bis zu 3 Kohlenstoffatomen enthaltende Alkoxygruppe bedeutet, bei Temperaturen zwischen 0 und 120°C durch Behandlung mit wässerigen Mineralsäuren hydrolysiert wird oder

c) o-Nitrophenylthioessigsäuren der allgemeinen Formel VI,

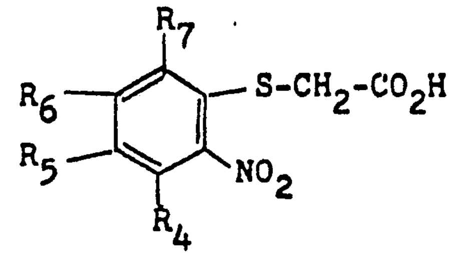
,(VI)

in der $R_4$, $R_5$, $R_6$ und $R_7$ wie eingangs definiert sind, mit Acetanhydrid zu den 3-Acetyl-2(3H)-benzothiazolonen der allgemeinen Formel VII,

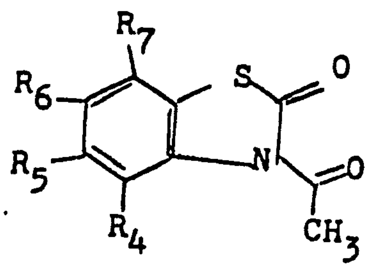
,(VII)

cyclokondensiert und anschließend die Acetylgruppe mittels Basen oder Säuren bei Temperaturen zwischen 0 und 120°C abgespalten wird oder

d) ein 2(3H)-Benzothiazolthion der allgemeinen Formel VIII,

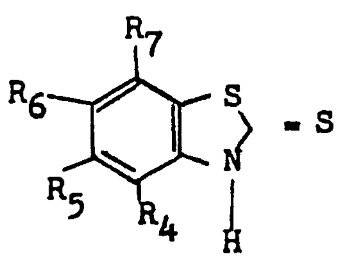
,(VIII)

in der $R_4$, $R_5$, $R_6$ und $R_7$ die oben angegebenen Bedeutungen besitzen, bei Temperaturen zwischen -10 und +30°C oxidiert

und zu einem 2(3H)-Benzothiazolon der allgemeinen Formel I mit wäßrigen Mineralsäuren bei Temperaturen zwischen 40 und 100°C hydrolysiert wird, wobei gegebenenfalls auch ein Alkalisalz eines 2(3H)-Benzothiazolthions der allgemeinen Formel VIII zunächst mit Alkyljodiden, Dialkylsulfaten oder Benzylhalogeniden zunächst zu einem 2-substituierten Benzothiazolon der allgemeinen Formel IXa,

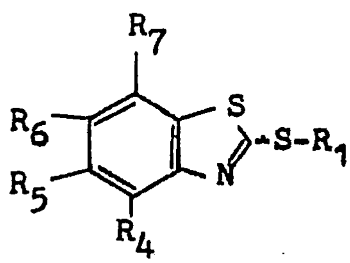

(IXa)

in der $R_4$, $R_5$, $R_6$ und $R_7$ wie eingangs definiert sind, und $R_1$ einen niederen Alkylrest mit 1 bis 3 Kohlenstoffatomen oder einen Benzylrest bedeutet, umgesetzt wird und dieses anschließend zu einem Sulfon der allgemeinen Formel IXb,

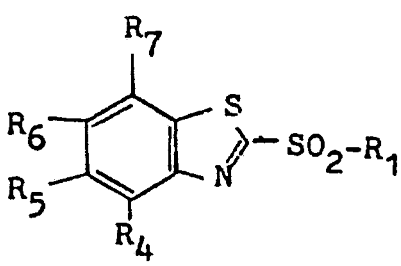

,(IXb)

oxidiert und letztere Verbindung mit wäßrigen Mineralsäuren bei Temperaturen zwischen 40 und 100°C hydrolysiert wird oder

e) zur Herstellung einer Verbindung der allgemeinen Formel I, in der $R_6$ die Dimethylaminogruppe und $R_4$, $R_5$ und $R_7$ Wasserstoffatome bedeuten, das 6-Amino-2(3H)-benzothiazolon mit Formaldehyd und Ameisensäure bei Temperaturen zwischen 60 und 100°C methyliert wird.

8) Verfahren gemäß Anspruch 7d, dadurch gekennzeichnet, daß die Oxidation einer Verbindung der allgemeinen Formel VIII mit bis zu 3 Mol Brom oder Chlor, mit mindestens 3 Mol Wasserstoffperoxid oder mindestens 2 Mol eines Alkalipermanganates pro Mol der zu oxidierenden Verbindungen der allgemeinen Formel VIII in wässerig alkalischem Medium durchgeführt wird und bei Verwendung von Kaliumpermanganat zunächst eine Sulfonsäure der allgemeinen Formel VIIIa

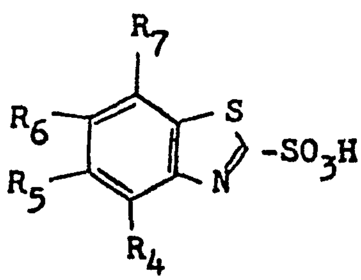

,(VIIIa)

entsteht, die anschließend durch Erwärmen auf 40 bis 100°C in Gegenwart wäßriger Mineralsäuren in eine Verbindung der allgemeinen Formel I übergeführt wird.

Patentansprüche für Österreich: Case 5/791

1) Verfahren zur Herstellung von neuen 2(3H)-Benzothiazolonen der allgemeinen Formel I

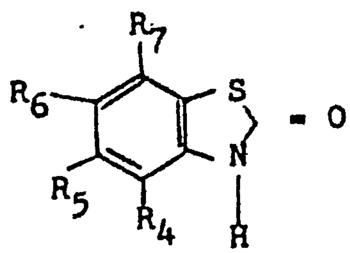

,(I)

in der

entweder

$R_4$ die Methylgruppe, $R_6$ die Methoxygruppe, $R_5$ und $R_7$ Wasserstoffatome oder

$R_4$ und $R_6$ jeweils die Methylgruppe, $R_5$ und $R_7$ Wasserstoffatome oder

$R_4$ die Methoxygruppe, $R_5$, $R_6$ und $R_7$ Wasserstoffatome oder

$R_4$ die Methoxygruppe, $R_5$ das Chloratom, $R_6$ und $R_7$ Wasserstoffatome oder

$R_5$ und $R_6$ zusammen die Methylendioxygruppe, $R_4$ und $R_7$ Wasserstoffatome oder

$R_7$ die Methylgruppe, $R_4$, $R_5$ und $R_6$ Wasserstoffatome oder

$R_5$ und $R_6$ jeweils die Methylgruppe, $R_4$ und $R_7$ Wasserstoffatome oder

$R_6$ und $R_7$ jeweils die Methylgruppe, $R_4$ und $R_5$ Wasserstoffatome oder

$R_6$ die Dimethylaminogruppe, $R_4$, $R_5$ und $R_7$ Wasserstoffatome oder

$R_5$ das Chloratom, $R_6$ die Methoxygruppe, $R_4$ und $R_7$ Wasser-

stoffatome oder $R_4$ und $R_6$ jeweils die Methoxygruppe, $R_5$ und $R_7$ Wasserstoffatome oder $R_5$ die Methoxygruppe, $R_6$ die Methylgruppe, $R_4$ und $R_7$ Wasserstoffatome oder $R_5$ und $R_6$ zusammen die Ethylendioxygruppe, $R_4$ und $R_7$ Wasserstoffatome oder $R_6$ und $R_7$ zusammen die Ethylendioxygruppe, $R_4$ und $R_5$ Wasserstoffatome bedeuten,

dadurch gekennzeichnet, daß

a) entweder ein 2-Aminothiophenol der allgemeinen Formel II,

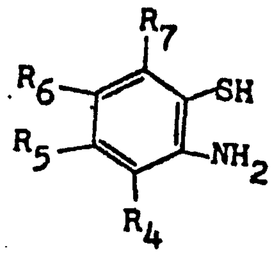

,(II)

in der $R_4$, $R_5$, $R_6$ und $R_7$ wie oben definiert sind, mit einem Kohlensäurederivat der allgemeinen Formel III,

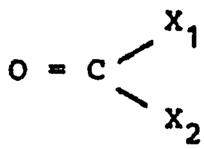

,(III)

in der $X_1$ und $X_2$ gleich oder voneinander verschieden sind und Halogenatome, die 1-Imidazolyl-, 1,2,4-Triazol-4-yl- oder die 1,2,4-Triazol-1-ylgruppe, eine Alkoxygruppe mit 1 bis 10 Kohlenstoffatomen, die Phenoxy- oder p-Tolyloxygruppe, die Benzyloxy- oder Phenethyloxygruppe oder zusammen ein Schwefelatom bedeuten, in einem Lösungsmittel bei Temperaturen zwischen 0 und 150°C umgesetzt wird

oder eine Verbindung der allgemeinen Formel II mit einem Chlorkohlensäureester der allgemeinen Formel IIIa,

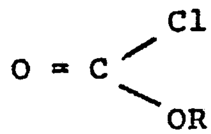 (IIIa)

in der R einen Alkylrest mit 1 bis 10 Kohlenstoffatomen oder die Phenyl-, 4-Methylphenyl-, Phenylmethyl- oder 2-Phenylethylgruppe bedeutet, zur Reaktion gebracht wird, wobei zunächst Verbindungen der allgemeinen Formel IV

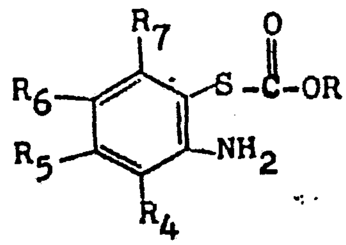 ,(IV)

in der R, $R_4$, $R_5$, $R_6$ und $R_7$ wie oben definiert sind, entstehen, die anschließend in Gegenwart saurer Katalysatoren bei Temperaturen zwischen 20 und 150°C zu den Verbindungen der allgemeinen Formel I cyclisiert werden, oder

b) ein in 2-Stellung durch Chlor, Brom oder eine Alkoxygruppe substituiertes Benzothiazol der allgemeinen Formel V,

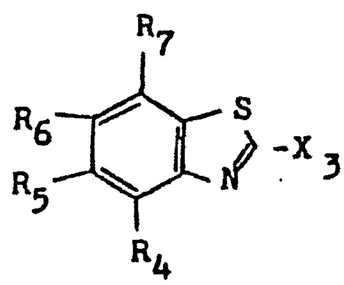 ,(V)

in der $R_4$, $R_5$, $R_6$ und $R_7$ wie oben definiert sind und $X_3$ ein Chlor- oder Bromatom oder eine bis zu 3 Kohlenstoffatomen enthaltende Alkoxygruppe bedeutet, bei Temperaturen zwischen 0 und 120°C durch Behandlung mit wässerigen Mineralsäuren hydrolysiert wird oder

c) o-Nitrophenylthioessigsäuren der allgemeinen Formel VI,

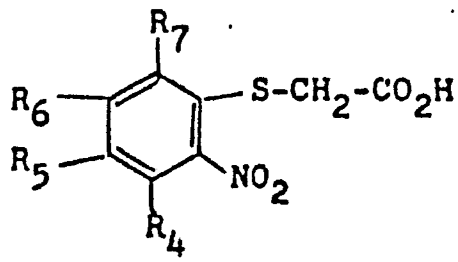

,(VI)

in der $R_4$, $R_5$, $R_6$ und $R_7$ wie eingangs definiert sind, mit Acetanhydrid zu den 3-Acetyl-2(3H)-benzothiazolonen der allgemeinen Formel VII,

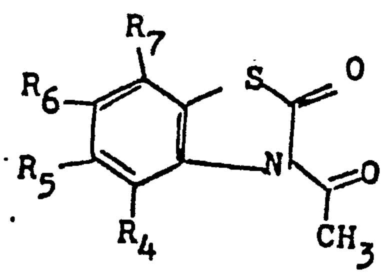

,(VII)

cyclokondensiert und anschließend die Acetylgruppe mittels Basen oder Säuren bei Temperaturen zwischen 0 und 120°C abgespalten wird oder

d) ein 2(3H)-Benzothiazolthion der allgemeinen Formel VIII,

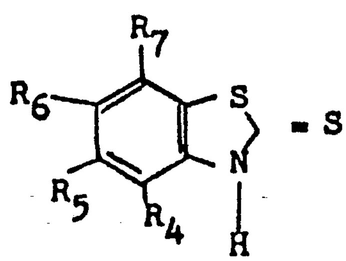

,(VIII)

in der $R_4$, $R_5$, $R_6$ und $R_7$ die oben angegebenen Bedeutungen besitzen, bei Temperaturen zwischen -10 und +30°C oxidiert

und zu einem 2(3H)-Benzothiazolon der allgemeinen Formel I mit wäßrigen Mineralsäuren bei Temperaturen zwischen 40 und 100°C hydrolysiert wird, wobei gegebenenfalls auch ein Alkalisalz eines 2(3H)-Benzothiazolthions der allgemeinen Formel VIII zunächst mit Alkyljodiden, Dialkylsulfaten oder Benzylhalogeniden zunächst zu einem 2-substituierten Benzothiazolon der allgemeinen Formel IXa,

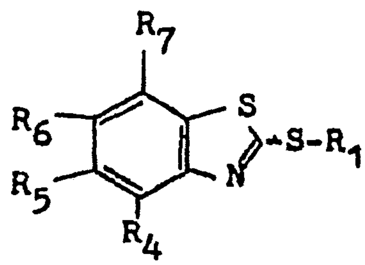

(IXa)

in der $R_4$, $R_5$, $R_6$ und $R_7$ wie eingangs definiert sind, und $R_1$ einen niederen Alkylrest mit 1 bis 3 Kohlenstoffatomen oder einen Benzylrest bedeutet, umgesetzt wird und dieses anschließend zu einem Sulfon der allgemeinen Formel IXb,

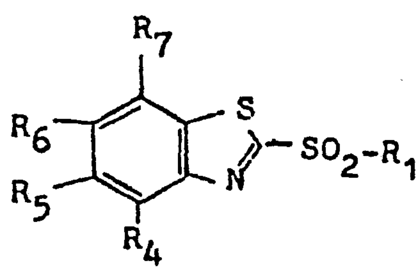

,(IXb)

oxidiert und letztere Verbindung mit wäßrigen Mineralsäuren bei Temperaturen zwischen 40 und 100°C hydrolysiert wird oder

e) zur Herstellung einer Verbindung der allgemeinen Formel I, in der $R_6$ die Dimethylaminogruppe und $R_4$, $R_5$ und $R_7$ Wasserstoffatome bedeuten, das 6-Amino-2(3H)-benzothiazolon mit Formaldehyd und Ameisensäure bei Temperaturen zwischen 60 und 100°C methyliert wird.

2) Verfahren gemäß Anspruch 1a, dadurch gekennzeichnet, daß die Umsetzung mit einer Verbindung der allgemeinen Formel III in Gegenwart einer anorganischen oder organischen Base und eines Lösungsmittels bei Temperaturen zwischen 20°C und der Siedetemperatur des Reaktionsgemisches erfolgt.

3) Verfahren gemäß Anspruch 1a , dadurch gekennzeichnet, daß die Cyclisierung von Verbindungen der allgemeinen Formel IV durch Mineralsäuren in inerten organischen Lösungsmitteln erfolgt.

4) Verfahren gemäß Anspruch 1b, dadurch gekennzeichnet, daß die Reaktion in Gegenwart inerter, mit Wasser mischbarer Lösungsmittel durchgeführt wird.

5) Verfahren gemäß Anspruch 1c, dadurch gekennzeichnet, daß die Cyclokondensation zu Verbindungen der allgemeinen Formel VII in Gegenwart von Alkaliacetaten oder von tertiären organischen Basen durchgeführt wird und die anschließende Hydrolyse mit Säuren oder Basen in Gegenwart von mit Wasser mischbaren Cosolventien erfolgt.

6) Verfahren gemäß Anspruch 1d, dadurch gekennzeichnet, daß die Oxidation einer Verbindung der allgemeinen Formel VIII mit bis zu 3 Mol Brom oder Chlor, mit mindestens 3 Mol Wasserstoffperoxid oder mindestens 2 Mol eines Alkalipermanganates pro Mol der zu oxidierenden

Verbindungen der allgemeinen Formel VIII in wässerig alkalischem Medium durchgeführt wird und bei Verwendung von Kaliumpermanganat zunächst eine Sulfonsäure der allgemeinen Formel VIIIa

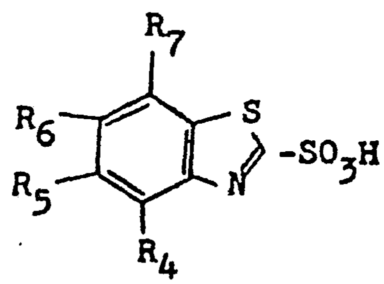
, (VIIIa)

entsteht, die anschließend durch Erwärmen auf 40 bis 100°C in Gegenwart wäßriger Mineralsäuren in eine Verbindung der allgemeinen Formel I übergeführt wird.

7) Verfahren gemäß Anspruch 1d, dadurch gekennzeichnet, daß ein 2-substituiertes Benzothiazolon der allgemeinen Formel IXa mit Kaliumpermanganat in alkalischer Lösung oder in Eisessig zu einem Sulfon der allgemeinen Formel IXb oxidiert und letzteres in Gegenwart von mit Wasser mischbaren Cosolventien mittels wäßriger Mineralsäuren in eine Verbindung der allgemeinen Formel I übergeführt wird.

8) Verfahren gemäß Anspruch 1e, dadurch gekennzeichnet, daß Formaldehyd und Ameisensäure jeweils in einer Menge von mindestens 2 Mol pro 1 Mol 6-Amino-2(3H)-benzothiazolon eingesetzt werden.

Europäisches Patentamt

# EUROPÄISCHER RECHERCHENBERICHT

0039818

Nummer der Anmeldung
EP 81 10 3152

| EINSCHLÄGIGE DOKUMENTE | | | KLASSIFIKATION DER ANMELDUNG (Int Cl ³) |
|---|---|---|---|
| Kategorie | Kennzeichnung des Dokuments mit Angabe, soweit erforderlich, der maßgeblichen Teile | betrifft Anspruch | |
| | CHEMICAL ABSTRACTS, Band 81, Nr. 23, 9. Dezember 1974, Seite 521, Nr. 152075p Columbus, Ohio, U.S.A. A.F. ABOULEZZ et al.: "Conversion of (o-nitrophenylthio)acetic acids to benzothiazolones" & EGYPT. J. CHEM. 1973, 16(4), 355-359 * Zusammenfassung * -- | 1,7 | C 07 D 277/68<br>513/04<br>A 61 K 31/425 |
| | CHEMICAL ABSTRACTS, Band 45, Nr. 16, 25. August 1951, Spalte 7109 b-i Columbus, Ohio, U.S.A. L. TORIZO TAKAHASHI et al.: "Syntheses of heterocyclic compounds of nitrogen" & J. PHARM. SOC. JAPAN 71, 41-44 1951 * Zusammenfassung * -- | 1 | RECHERCHIERTE SACHGEBIETE (Int. Cl ³)<br>C 07 D 277/68<br>513/04<br>A 61 K 31/425 |
| | GB - A - 830 902 (J.R. GEIGY) * Seite 1 * -- | 1,7 | |
| | FR - A - 2 143 362 (FARBWERKE HOECHST) * Anspruch 1 * -- | 1,7 | KATEGORIE DER GENANNTEN DOKUMENTE<br>X: von besonderer Bedeutung<br>A: technologischer Hintergrund<br>O: nichtschriftliche Offenbarung<br>P: Zwischenliteratur<br>T: der Erfindung zugrunde liegende Theorien oder Grundsätze<br>E: kollidierende Anmeldung<br>D: in der Anmeldung angeführtes Dokument<br>L: aus andern Gründen angeführtes Dokument<br>&: Mitglied der gleichen Patentfamilie, übereinstimmendes Dokument |
| | CHEMICAL ABSTRACTS, Band 42, Nr. 9, 10. Mai 1948, Spalte 3073 e-h Columbus, Ohio, U.S.A. B.L. FREEDLANDER et al.: "Chemotherapy of experimental tuberculosis with benzothiazole deriva- ./. | 1,5 | |

X Der vorliegende Recherchenbericht wurde für alle Patentansprüche erstellt.

| Recherchenort | Abschlußdatum der Recherche | Prüfer |
|---|---|---|
| Den Haag | 05-08-1981 | HENRY |

0039818

Nummer der Anmeldung

EP 81 10 3152

-2-

Europäisches Patentamt

# EUROPÄISCHER RECHERCHENBERICHT

| EINSCHLÄGIGE DOKUMENTE | | | KLASSIFIKATION DER ANMELDUNG (Int.Cl. 3) |
|---|---|---|---|
| Kategorie | Kennzeichnung des Dokuments mit Angabe, soweit erforderlich, der maßgeblichen Teile | betrifft Anspruch | |
| | tives"<br>& PROC. SOC. EXPTL. BIOL. MED. 66, 362-365, 1947<br>* Zusammenfassung *<br>-- | | |
| P | EP - A - 0 022 213 (DR. KARL THOMAE)<br>* Ansprüche *<br>---- | 1,5,6 | |
| | | | RECHERCHIERTE SACHGEBIETE (Int. Cl. 3) |